# EUROPEAN PATENT SPECIFICATION

(11) **EP 0 467 173 B1**
(45) Date of publication and mention of the grant of the patent: **08.11.1995**
(21) Application number: 91111239.9
(22) Date of filing: 05.07.1991
(51) Int. Cl.: A61M 5/32

(54) **Disposable safety syringe**
Sicherheitsspritze zum einmaligen Gebrauch
Seringue de sécurité à usage unique

(30) Priority: 19.07.1990 IT 2097990; 09.01.1991 IT GE910008
(43) Date of publication of application: 22.01.1992
(73) Proprietor: Righi, Nardino, I-20093 Cologno Monzese (IT); Rossi, Roberto, I-20151 Milano (IT)
(72) Inventor: Righi, Nardino, I-20093 Cologno Monzese (IT); Rossi, Roberto, I-20151 Milano (IT)
(74) Representative: Porsia, Bruno

(56) References cited:
- EP-A- 0 307 367
- EP-A- 0 405 039
- FR-A- 1 567 778
- FR-A- 2 650 187
- GB-A- 2 197 792
- US-A- 4 927 018
- US-A- 4 927 416
- US-A- 4 935 015

## Description

The invention relates to a syringe that is not reusable, and therefore constitutes a safety syringe.

More particularly, the invention relates to a disposable safety syringe according to the preamble of claim 1 comprising:
a) a barrel,
b) an injection needle that is attached to a needle-carrying member disengageably fitted in, and/or on, the fore end of the barrel,
c) a plunger that is longitudinally slidable in the barrel from an utmost retracted syringe-filling position to a forwardmost syringe-emptying position, and is fitted with a manually drivable stem protruding from the rear end of the barrel,
d) a protective sleeve that is slidably fitted on the outside of the barrel, so as to be movable from a retracted rest position in which the needle is exposed, into an advanced safety position in which the protective sleeve extends all around the needle so as to entirely cover the needle,
e) clamping means comprising elastically flexible clamping tongues and hook-like clamping teeth that are provided at the rear free ends of said elastically flexible clamping tongues extending in the longitudinal direction in the protective sleeve, and being made of one piece therewith, and the said clamping teeth cooperate with a matching retaining rim on the rear end of the barrel, the protective sleeve, in its retracted position being fastened to the barrel, by said clamping teeth engaged with said retaining rim
f) releasing means for disengaging the clamping teeth from the retaining rim on the rear end of the barrel, at the end, or almost at the end, of the forward stroke of the plunger, comprising a disc-shaped pusher member that is secured to the rear end section extending out of the barrel of the plunger stem and cooperates with the clamping teeth, the said releasing means thus unfastening the protective sleeve from barrel and allowing the protective sleeve to be moved in its advanced safety position,
g) a spring that is fitted between the barrel and the protective sleeve, and is adapted for moving the protective sleeve from its retracted rest position into its advanced safety position,
h) locking means that are provided for automatically retaining the protective sleeve in its advanced safety position, in a not retractil manner to the barrel.
   A syringe of thus type is known from the European Patent Application 0 307 367.
   In this known syringe, the protective sleeve having been snapped by the spring into its advanced safety position, can be torn away and slipped forwardly off the barrel, by breaking or forcing apart the clamping tongues having their clamping teeth engaged with the barrel body.
   Moreover, in this known syringe the releasing pusher member secured to the rear end section of the pusher stem, cooperates with the clamping teeth by means of a split ring received in an annular groove in the inner wall of the barrel, which also prevents the plunger that has been driven into its forwardmost syringe-emptying position, from being drawn back into its retracted syringe-filling position. The split ring has to be fitted beforehand on the plunger stem, at the interior of the barrel, opposite to the releasing pusher member, which in turn has to be located opposite to the annular groove in the inner wall of the barrel.
   The object of the invention is to improve the aforementioned known syringe by definitely preventing the syringe needle from becoming exposed after the first injection, thus eliminating any risk of infection, as well as by the provision of a remarkably simple construction of the syringe, which results in a reduced expenditure for making and assembling the syringe.
   This problem is solved by the invention -- in a syringe of the type as specified at the outset -- by the combination of the following features, as recited in the characterising portion of claim 1:
i) two diametrically opposite, slip back-preventing teeth are provided at the rear end of barrel, which teeth can be caused to elastically diverge radially outwardly from each other, and have their facing inward sides formed with slanted abutment faces, the said slip back-preventing teeth being caused to cooperate with the pusher member secured to the rear end section of the stem of plunger, in such a manner that the said teeth when being elastically flexed in the outward direction, will allow the pusher member to pass therebetween, and will be then engaged with the rear side of the pusher member, so that the pusher member becomes locked together with the stem of plunger, in the forwardmost position of the plunger stroke, whereby any backward displacement of the pusher member and the stem is prevented,
j) the slip back-preventing teeth are formed by means of cuts made in the sidewall of a boxlike head which is integrally provided at the rear end of the barrel, and the clamping tongues protruding from the rear end of the protective sleeve, extend by their free ends formed with the hook-like clamping teeth and slanted abutment faces, into the said boxlike head, by being passed through respective apertures formed in the bottom of the said head, the retaining rims at the rear end of the barrel being provided by the edges of said apertures with which the said clamping teeth are engaged, and the boxlike head being formed with an opening at its rear side for the disc-shaped releasing pusher member secured to the rear end section of the plunger stem, to get into the said head,
k) removable safety means are provided for closing at least partly the opening at the rear end of the boxlike head and for preventing the pusher member from getting into the said head,
l) the locking means for automatically retaining the protective sleeve in its advanced safety position to the barrel, lock the protective sleeve in both senses with the needle-carrying member.

The advantages as afforded by the invention, are first of all obtained thanks to the feature that, should the protective sleeve be forcibly slipped forwardly off the barrel after an injection, also the needle-carrying member with the needle would be detached from the barrel and slipped forward, along with the protective sleeve. Since the needle-carrying member is locked within the protective sleeve in both senses of the axial direction, the needle is kept locked within the protective sleeve even after the protective sleeve having been torn off, so that the needle is by no means accessible. Thus, any risk that a person may get pricked and infected by the syringe needle, is eliminated. At the same time, the plunger is prevented from being drawn back into its retracted syringe-filling position by the provision of means of a simple construction, made of one piece with the barrel. No difficulty arises in assembling the said means, since the plunger can be conventionally inserted into the barrel, through the rear end thereof, and both the barrel, on the one hand, and the plunger with its stem and the releasing pusher member, on the other hand, can be made in one piece. Such a simplified construction, according to the invention, of the means for preventing the plunger from being drawn back into its retracted syringe-filling position, is advantageously crowned with the removable safety means which serve to prevent the plunger stem from being inadvertently pushed forward from its utmost retracted initial position, into a position in which the releasing pusher member is locked by the slip back-preventing teeth,

Also other embodiments of the invention are characterized in the dependent claims, and the advantages attained thereby will become clearly apparent from the following disclosure of some embodiments shown in the accompanying drawings, in which:
Figure 1 is an exploded perspective view of a first embodiment of the syringe according to the invention.
Figure 2 is a longitudinal sectional view of the syringe according to Figure 1, with the protective sleeve lying in its retracted position.
Figure 3 shows the syringe according to Figure 2, viewed in the direction of arrow III in this latter Figure, and with some parts in section.
Figure 4 is a same view of the syringe as in Figure 3, however with the protective sleeve lying in its advanced safety position.
Figure 5 is a longitudinal sectional view of the syringe, with the protective sleeve having been separated from the syringe together with the needle-carrying member and the needle, when an attempt is made of re-using the syringe.
Figure 6 is a perspective view showing the way of holding the syringe according to Figures 1 to 5, at the time of an injection.
Figure 7 is a longitudinal sectional view in an enlarged scale, showing a detail of the syringe according to Figure 4.
Figure 8 is an exploded perspective view showing a second embodiment of the syringe according to the invention.
Figure 9 is a longitudinal sectional view in an enlarged scale showing the rear end of the syringe according to Figure 8, with the protective sleeve lying in its retracted rest position.
Figure 10 is a longitudinal sectional view in the same scale as in Figure 9, showing the fore end of the protective sleeve of the syringe according to Figure 8.
Figure 11 is a view with parts in section, showing the rear end of the syringe according to a third embodiment of the invention, with the protective sleeve lying in its advanced safety position.
Figures 12 and 13 are elevational part-sectional views showing the rear end of the syringe according to Figure 11, with the protective sleeve in its retracted rest position (Figure 12), and at the time when the said sleeve is being released from the syringe barrel (Figure 13).
Figure 14 is a cross-sectional view taken on line XIV-XIV in Figure 12.
Figures 15 and 16 are prespective views showing two details of the syringe according to Figures 11 to 14.

In the following disclosure and in the annexed Claims, by the expression "syringe fore end" we mean the end of the syringe that is fitted with the injection needle, and the rear end of the syringe is the end thereof lying opposite to the needle. Also, all the syringe members, but for the needle, are preferably made from a suitable plastics material, or the like, unless when it is differently specified.

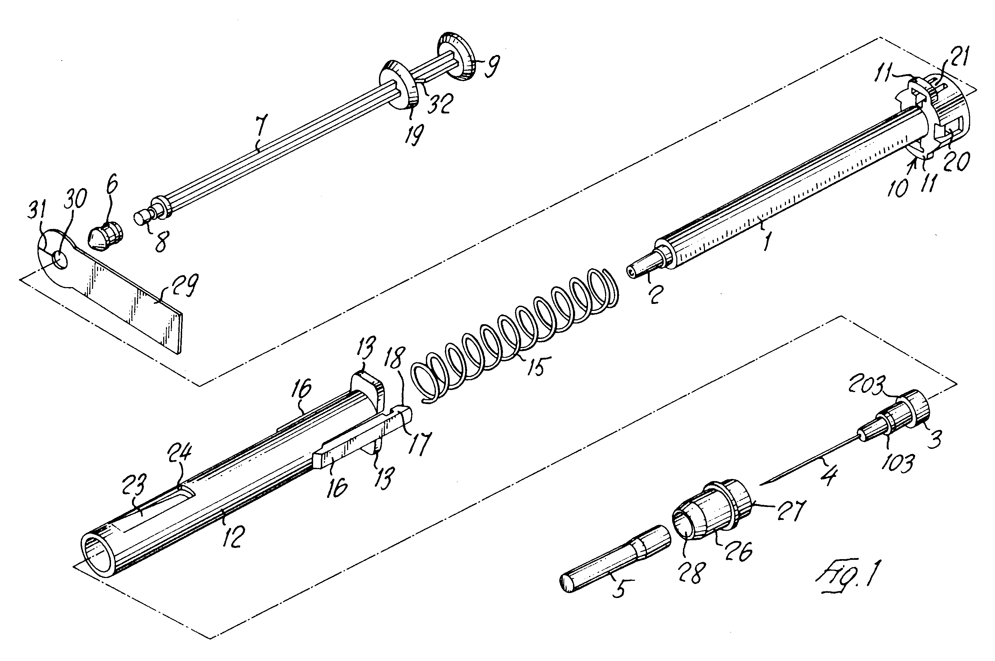

Referring to the embodiment shown in Figures 1 to 7, the disposable safety syringe comprises a barrel 1 with a conical forward end 2 on which the needle-carrying member 3 is fitted and is held by friction by means of a matching conical hole formed at the rear end thereof, the rear end of needle 4 being firmly incorporated in the said hole. The needle 4 is normally protected by a needle-covering cap 5 disengageably fitted on the fore end of the needle-carrying member 3 so as to be caused to abut against an undercut 103 provided therein. In barrel 1 a plunger 6 is axially slidable in a fluid-tight manner, and the head 8 of the manually drivable stem 7 is engaged in the said plunger. The rear end section of stem 7 extends backwardly out of barrel 1, and is provided with a knob 9 on the rear end thereof. Preferably, the stem 7 has in cross-section a non-circular shape, and is for example, T-like or X-like shaped. The rear end portion of barrel 1 is so enlarged that a substantially cylindrical boxlike head 10 is formed, and is provided with two diametrically opposite tabs 11 radially extending from its periphery.

A protective sleeve 12 is fitted on the outside of barrel 1 so as to be longitudinally slidable thereon. The rear end of the protective sleeve 12 is formed with two diametrically opposite tabs 13 which extend radially from the periphery thereof, and are like the tabs 11 of barrel 1. A helical spiral spring 15 of metal is interposed between an inward step 14, provided in the protective sleeve 12 at a distance from its rear ends and the boxlike head 10 of barrel 1.

The protective sleeve 12 is provided at its rear end with two clamping tongues 16 which are made of one piece therewith, and are formed therein at two diametrically opposite locations. Each clamping tongue 16 extends lengthwise of the protective sleeve 12, at a longitudinal aperture 47 made therein. The forward end of the clamping tongue 16, i.e., the end thereof which is turned toward the needle 4, is integral with the protective sleeve 12, and its rear end extends beyond the rear end of the protective sleeve 12, and protrudes from the rear end thereof. Each clamping tongue 16 can be elastically flexed outwardly in the radial direction, and at its rear end is formed with a hook-like clamping tooth 17 which is turned radially inwardly. Each clamping tongue 16 is further provided at the rear end of its clamping tooth 17 with a slanted abutment face 18 which is downwardly inwardly inclined, and is for cooperating with the correspondingly inclined peripheral side edge of a disc-shaped pusher member 19 secured to the stem 7 of plunger 6 at the rear side of the boxlike head 10 of barrel 1, which is opposite to the clamping tongues 16.

The boxlike head 10 at the rear end of barrel 1 is formed close to each clamping tongue 16, with an aperture 20 extending into the bottom and into the sidewall of the said boxlike head 10. Thus, each clamping tongue 16 is allowed to penetrate into the boxlike head 10 and to be engaged by its hook-like clamping tooth 17 with the bottom of the said head 10. By means of cuts made in the sidewall of the boxlike head 10 at the rear end of barrel 1, two diametrically opposite, slip back-preventing teeth 21 are formed, which are angularly offset by 90° from the two apertures 20 for the clamping tongues 16. The slip back-preventing teeth 21 can be caused to elastically diverge radially outwardly from each other, and have their facing inward sides formed with slanted abutment faces 22 which are downwardly inwardly inclined, and are for cooperating with the correspondingly inclined peripheral side edge of the disc-shaped pusher member 19.

In the fore part of the protective sleeve 12 two diametrically opposite locking tongues 23 are provided, which are formed in the protective sleeve 12 by means of cuts 24 made therein. Each locking tongue 23 extends in the longitudinal direction of the protective sleeve 12. The forward end of each locking tongue 23 is integral with the protective sleeve 12, and the rear end thereof is loose and tends to elastically flex radially inwardly. The rear free ends of the locking tongues 23 are for cooperating with a peripheral outward undercut 203 in the needle-carrying member 3. The protective sleeve 12 is formed in the direction of the syringe rear end, at a distance from the locking tongues 23, with an inward locking projection 25 which may be made in form of an annular undercut or may consist of the fore ends of a plurality of ribs (not shown) provided at the interior of the protective sleeve 12. The inward projection 25 for locking the protective sleeve 12, is for cooperating with the annular edge portion 303 of the rear end rim 303 of the needle-carrying member 3. The needle-carrying member 3 having been fittedly positioned on the fore end 2 of barrel 1, actually protrudes from the periphery of barrel 1 by the edge portion of its rear end rim, so that an outward annular step 303 is formed, which is associated with the inward locking projection 25 in the protective sleeve 12.

A tubular member 26 is engaged in the fore end of the protective sleeve 12, and is fastened thereto such as by glueing or by welding. The rear end rim of the said tubular member 26 forms a retaining inward annular projection 27 in the protective sleeve 12, and its front hole 28 is tapered relative to the inside diameter of the protective sleeve 12, and is only slightly greater than the outside diameter of the rear end portion of the needle-covering cap 5.

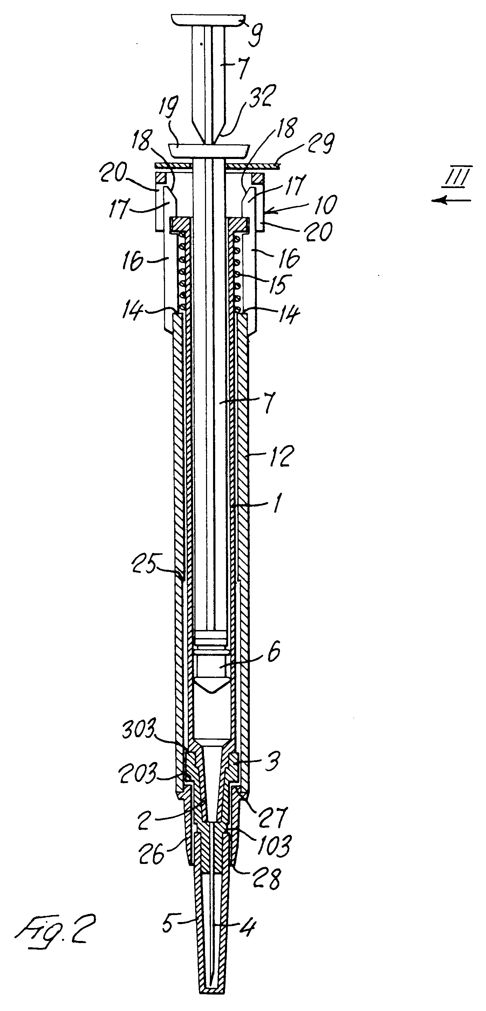

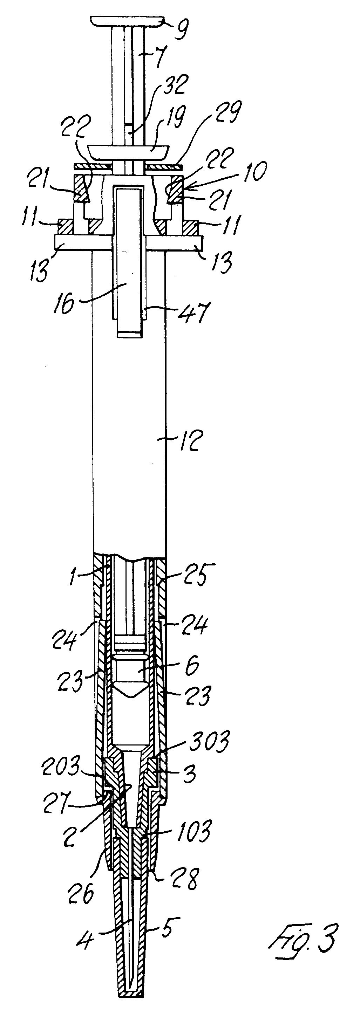

The syringe is sold to a user in the condition shown in Figures 2 and 3, in which the plunger 6 is slightly drawn back from its forwardmost position, in which the syringe is entirely discharged. In this condition of the syringe, the disc-shaped pusher member 19 is a little out of the boxlike head 10 at the rear end of barrel 1, and is prevented from getting into the said head by a tearable safety strip 29 of paper or paperboard, or the like. Through a hole 30 and a radial cut 31 the said strip 29 is threaded on the stem 7 of plunger 6 and is positioned between the disc-shaped pusher member 19 and the rim of the opening at the rear end side of the said boxlike head 10. The protective sleeve 12 lies in its retracted rest position in which the clamping tongues 16 are caused to penetrate through the apertures 20 into the boxlike head 10 of barrel 1. Thus, the hook-like clamping teeth 17 of the clamping tongues 16 are engaged with the bottom of the said head 10, whereby the protective sleeve 12 is fastened to the barrel 1. With the protective sleeve 12 lying in its retracted rest position, the spring 15 is loaded, that is, compressed, and the tabs 13 radially extending from the periphery of the protective sleeve 12, are caused to bear, or almost bear against the forward face of the corresponding tabs 11 provided on the boxlike head 10 of barrel 1. Moreover, when the protective sleeve 12 is in its retracted rest position, the tapered fore part 26 thereof extends substantially to the forward end of the needle-carrying member 3, so that the needle 4 is set free in an exposed condition when the needle-covering cap 5 will be forwardly disengaged. Concurrently, the protective sleeve 12 is caused to bear, or almost bear by its inward annular projection 27 formed by the rear end rim of the tubular member 26, backwardly against the outward annular undercut 203 in the needle-carrying member 3. In the above-disclosed condition of the syringe according to Figures 2 and 3, the plunger 6 can be freely retracted by drawing back its stem 7, so that the syringe barrel 1 will be filled as usual by sucking the to-be-injected liquid through the exposed needle 4.

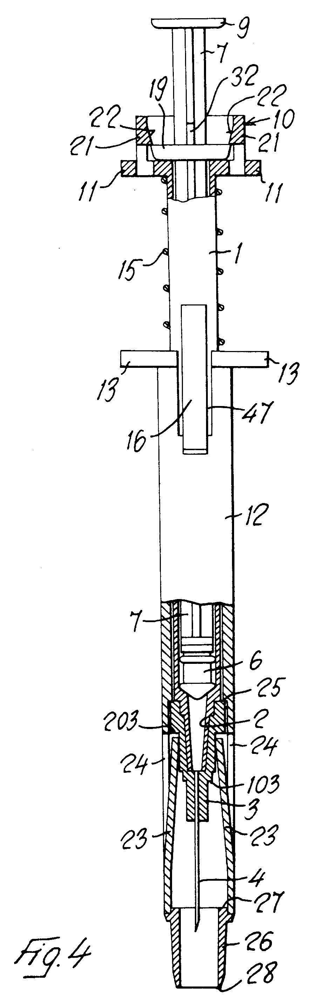

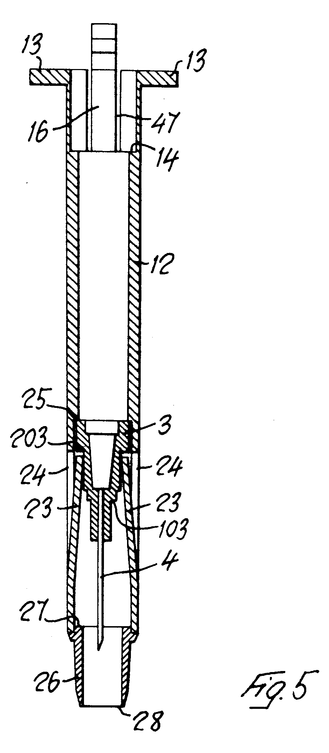

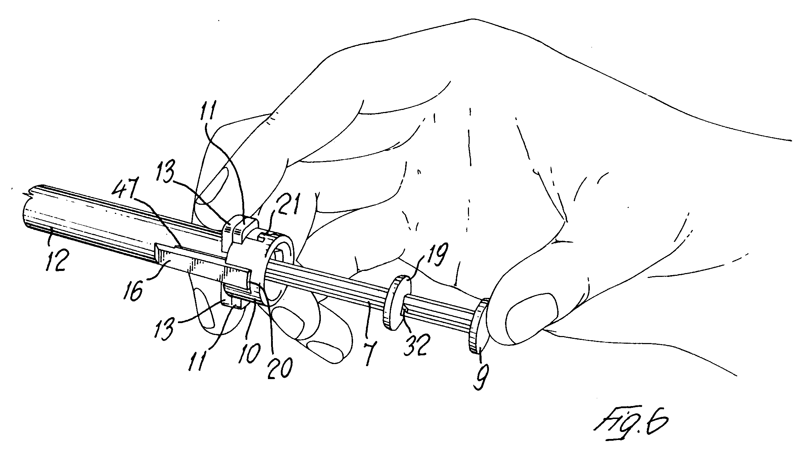

To be allowed to make an injection, the safety strip 29 has to be torn away, and the syringe has to be held as shown in Figure 6, with two fingers of a user's hand resting each on the front side of the respective tab 13 extending radially from the periphery of the rear end of the protective sleeve 12, and with the used's thumb placed on the knob 9 of the plunger stem 7 pressing forward the plunger 6. Toward the end of the plunger 6 foward stroke, the disc-shaped pusher member 19 is caused to penetrate into the barrel 1 boxlike head 10 from the rear end opening thereof, and by its peripheral side edge is caused to act on the slanted abutment faces 18 of the clamping tongues 16. Therefore, the clamping tongues 16 are caused to elastically diverge outwardly from each other, so that their hook-like clamping teeth 17 become disengaged from the bottom of the boxlike head 10 of barrel 1. The protective sleeve 12 is thus unfastened from barrel 1, but is still retained in its retracted rest position against the load of the pressure spring 15, until the syringe is held by the operator's hand, as disclosed by referring to Figure 6. Before and/or as the clamping tongues 16 are opened out as disclosed above, the disc-shaped pusher member 19 is caused to act by its peripheral side edge also upon the slanted abutment faces 22 of the slip back-preventing teeth 21, and to get thereover. The said teeth 21 are thus elastically opened out, so that they are caused to snappingly engage the top side of the disc-shaped pusher member 19. Therefore, on injecting, the pusher member 19 is locked in the boxlike head 10 of barrel 1 at the end or almost at the end of the forward stroke of plunger 6, between the bottom of the said head 10 and the slip back-preventing teeth 21, in such a position that the clamping tongues 16 are kept in their opened out condition, and the protective sleeve 12 is thus disengaged from the barrel 1, as shown in the upper part of Figure 4.

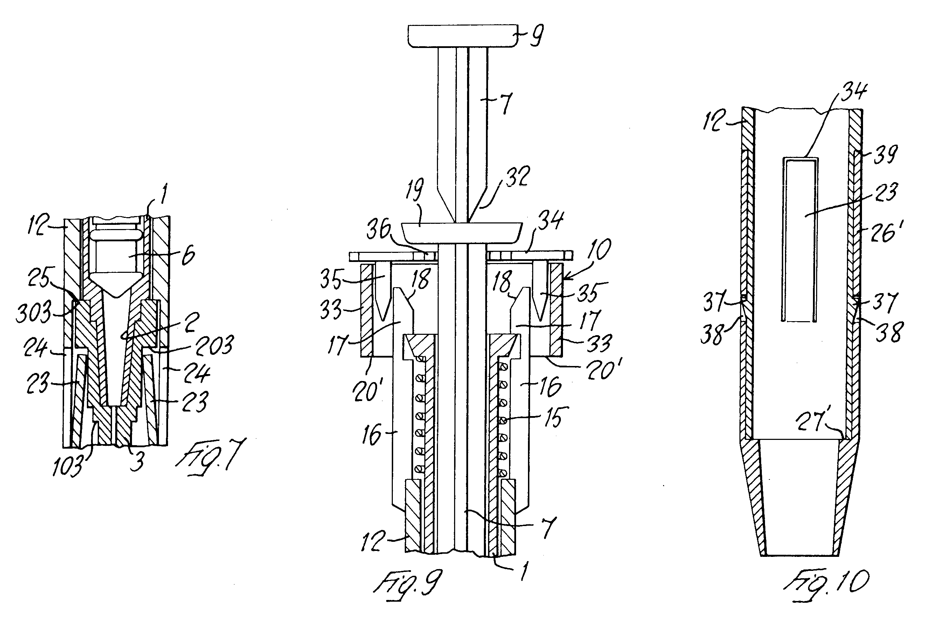

Once the injection has been made, the peripheral radial tabs 13 extening from the rear end of the protective sleeve 12 are released, so that the protecive sleeve which is now disengaged from the barrel 1, is moved forward by the spring 15, either gradually or snappingly into its advanced safety position shown in Figure 4. The protective sleeve lying in its advanced safety position, extends around the needle 4 so as to entirely cover the same. The protective sleeve 12 is at the same time caused to abut by its inward locking projection 25, backward against the annular step 303 formed by the edge portion of the rear end rim of the needle-carrying member 3, and the rear free ends of the locking tongues 23 are simultaneously caused to bear backward against the outward annular undercut 203 in the needle-carrying member 3, as more particularly shown in Figure 7. Thus, the protective sleeve lying in its advanced safety position, is unremovably locked to the barrel 1 in both senses of its longitudinal direction, i.e., either forward and backward, by means of the needle-carrying member 3. Should a person attempt to slip the protective sleeve 12 forwardly off the cylinder 1 when trying to re-use the syringe, the needle-carrying member 3 would be, at the most, disengaged from the conical forward end of barrel 1, so that the protective sleeve 12 would be detached from barrel 1 together with the needle-carrying member 3 that is inseparably shut in the protective sleeve 12, and so together with the needle 4 enclosed in the protective barrel 12, as shown in Figure 5. The protective sleeve 12 can be made with no problem and at a low cost from such a tough and strong plastics material that the protective sleeve could not be broken without damaging and rendering the needle-carrying member 3 and also the needle 4 unusable. Moreover, the fore part 26 of the protective sleeve 12 has such a narrow hole 28 and extends over such a long section beyond the pointed end of needle 4, that the finger of a person is prevented from reaching to the needle 4 from the fore end of the syringe.

The stem 7 of plunger 6 may be provided with an easily breakable weakened portion 32 at a point between the disc-shaped pusher member 19 and the knob 9 on the rear end of stem 7, preferably at a point being adjacent to the disc-shaped pusher member 19. Owing to the provision of this weakened portion, the rear end section of stem 7 will be broken when an attempt is made to pull back the plunger 6 for trying to re-use the syringe, once the disc-shaped pusher member 19 has been locked in the boxlike head 10 of barrel 1 by the slip back-preventing teeth 21.

The protective sleeve 12 can be slidably but non-rotatably fitted on the barrel 1 with the aid of simple means known to those skilled in the art, such as by an inward projection in sleeve 12, slidably engaged in a longitudinal groove in the outward side of barrel 1, or vice-versa.

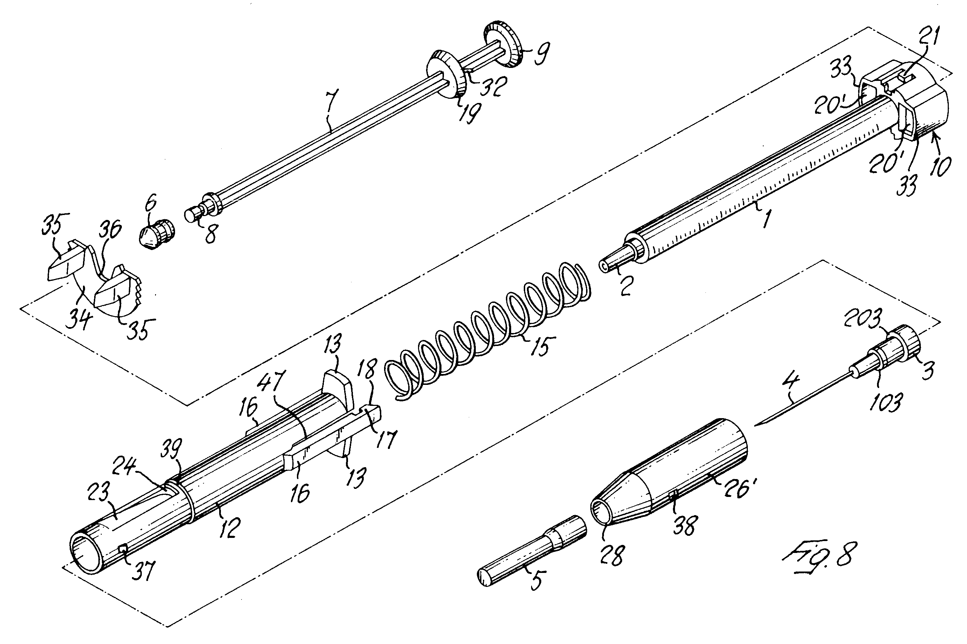

The embodiment of the syringe according to Figures 8, 9, and 10, substantially corresponds to the emdbodiment as disclosed above by referring to the Figures 1 to 7, like parts being designated by the same reference numerals.

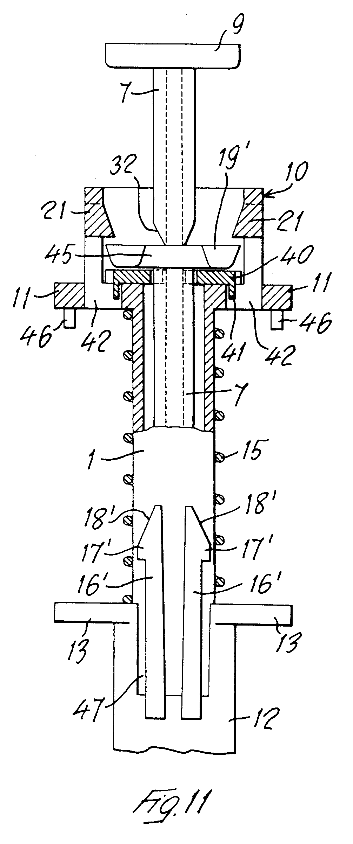

However, in the modified embodiment according to Figures 8 to 10, the boxlike head 10 at the rear end of barrel 1 has a sidewall 33 radially covering from the outside the rear free ends forming the hook-like clamping teeth 17 of the clamping tongues 16, which have been caused to get into the said boxlike head 10 through the apertures 20' in the bottom thereof. The removable safety means for initially preventing the disc-shaped pusher member 1 from entering into the boxlike head 10 at the rear end of barrel 1, consist of a substantially part-circular small cover member 34 formed with a central opening 36 for allowing the stem 7 to pass therethrough, which is provided in place of the tearable strip 29 of paper or paperboard. The small cover member 34 is applied to the opening at the rear end side of the boxlike head 10 and, for this purpose, is formed at its front side which is turned toward the syringe fore end, with two diametrically opposite wedge-shaped teeth 35. The said teeth 35 which extend from the cover member 34, are introduced into the opening at the rear end side of the boxlike head 10 so as to be inserted between the two clamping tongues 16 which by means of their hook-like clamping teeth 17, are engaged with the bottom of the said boxlike head 10, and the respective portion of the sidewall 33 thereof, as shown in Figure 9. Thus, the two teeth 35 will hold the cover member 34 in position on the boxlike head 10 at the rear end of barrel 1, and at the same time will prevent the rear free ends of the clamping tongues 16 from being radially outwardly flexed and then from being disengaged from the said boxlike head 10. The thus applied cover member 34 partly closes the opening of the boxlike head 10 at the rear end of barrel 1, and therefore prevents the disc-shaped pusher member 19 from entering into the said head 10.

Before the syringe being used for an injection, the cover member 34 must be removed. Therefore, a syringe user has to grip the said cover member 34 with two fingers of its hand close to the preferably indented peripheral edge thereof, at two diametrically opposite positions, so as to axially disengage the said cover member 34 from the boxlike head 10 at the rear end of barrel 1, and to nextly slip the same radially off the stem 7 of plunger 6.

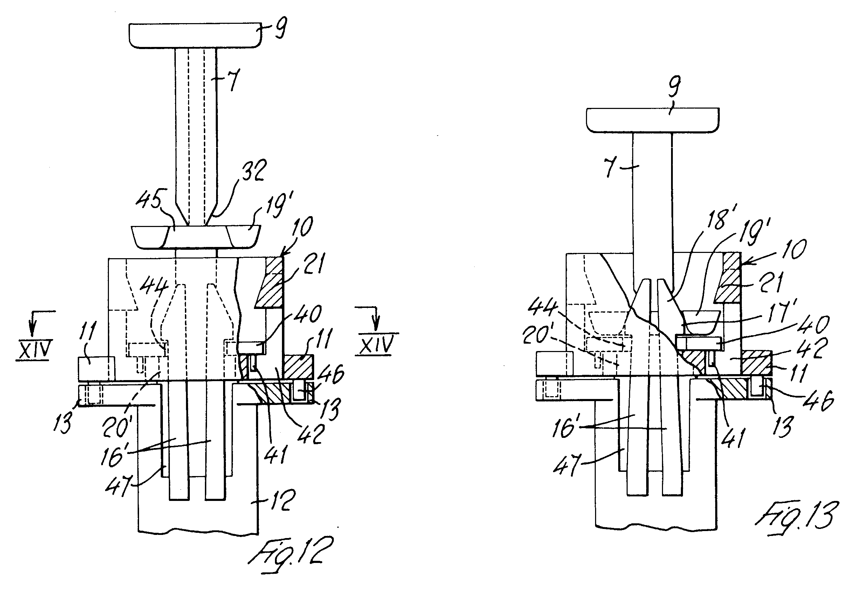

According to the embodiment shown in Figures 8, 9, and 10, the tubular member 26' forming the fore part of the protective sleeve 12, has an tapered front hole 28 which is only a little wider than the rear end portion of the needle-covering cap 5. The said tubular member 26' is threaded on the outside of the protective sleeve 12 and is snappingly engaged therewith in an unremovable manner. Therefore, the protective sleeve 12 is provided with a front end section of a reduced diameter, in which the locking tongues 23 are formed, and on which the tubular member 26' is fitted so as to be caused to abut against an outward annular undercut 39 formed in the protective sleeve 12. The tubular member 26' is locked in this position by two outward detents 37 which are provided in diameterically opposite positions on the outside of the protective sleeve 12, and are snappingly engaged into respective peripheral slots 38 in the tubular member 26'. With the tubular member 26' being in its engaged position, the said tubular member is caused to abut by an inward annular step 27' thereof, against the front end rim of the protective sleeve 12, as more particularly shown in Figure 10. The width of the internal annular step 27' is such that this step partly extends also into the protective sleeve 12, so that its radially inwardly edge portion forms the retaining annular projection which is for cooperating with the outward annular undercut 203 in the needle-carrying member 3.

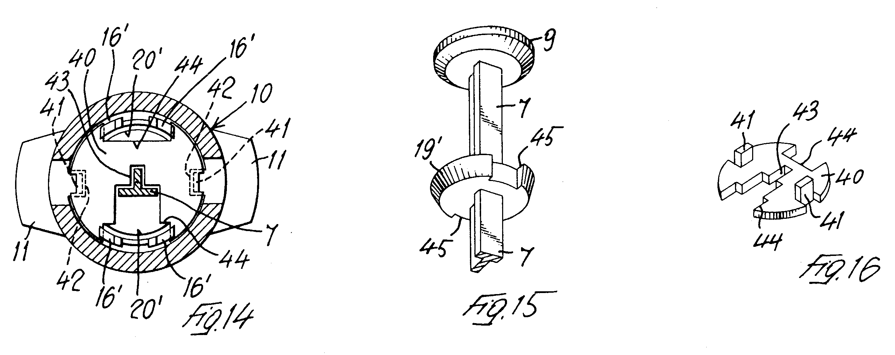

Also in Figures 11 to 16 showing a further modified embodiment of the syringe according to the invention, the already described parts are designated by the same reference numerals. A platelet 40, which is shown in upturned position in Figure 16, is arranged on the bottom of the boxlike head 10 of barrel 1, and by means of two diametrically opposite tongues 41 is engaged in the respective apertures 42 formed in the bottom of the said head 10. The platelet 40 is thus non-rotatably retained in the boxlike head 10 of barrel 1, and is caused to non-rotatably guide the stem 7 of plunger 6. the stem 7 is T-shaped in cross section, and by the leg of its T-shaped cross-section is passed through a relative narrow opening 43 in platelet 40. Instead of two diametrically opposite clamping tongues 16 as provided in the embodiments shown in Figures 1 to 10, two pairs of diametrically opposite clamping tongues 16' are provided in the syringe according to the modified embodiment shown in Figures 11 to 16, at respective longitudinal apertures 47 in the protective sleeve 12. The two clamping tongues 16' of each pair are arranged in facing relation along an arc, and in a substantially tangential plane, with their hook-like clamping teeth 17' being turned outwardly in opposite directions, and with their slanted abutment faces 18' being downwardly outwardly inclined. Each pair of clamping tongues 16' is caused to get into the boxlike head 10 at the rear end of barrel 1 through an aperture 20'in the bottom of the said head 10, and through a relative peripheral aperture 44 formed in platelet 40. The hook-like clamping teeth 17' of the clamping tongues 16' are each engaged with the edges of the relative peripheral aperture 44 in platelet 40. In register with each pair of clamping tongues 16', the disc-shaped pusher member 19' secured to the stem 7 of plunger 6, is formed with an opening consisting of a peripheral recess 45. On injecting, the free ends of the pairs of clamping tongues 16' come to be inserted at the end, or almost at the end of the forward stroke of plunger 6, into the peripheral recesses 45 in the disc-shaped pusher member 19' which is thus caused to cooperate with the slanted abutment faces 18' of the said pairs of clamping tongues 16', so that it elastically draws the two clamping tongues 16' of each pair near to each other, as more particularly shown in Figure 13. Thus, the hook-like clamping teeth 17' of the clamping tongues 16' become disengaged from the edges of the respective peripheral aperture 44 in platelet 40 at the bottom of the boxlike head 10 on the rear end of barrel 1, so that the protective sleeve 1 is unfastened from the barrel 1, and is moved forward by the bias of spring 15, as disclosed above by referring to Figures 1 to 7. At the time when the injection is terminated, the disc-shaped pusher member 19' becomes locked between the said platelet 40 and the slip-back preventing teeth 21 provided in the boxlike head 10, as more particularly shown in Figure 11. The diametrically opposite tabs 11 radially extending from the periphery of the boxlike head 10, are preferably fitted with pins 46 which are engaged in respective holes in tabs 13 radially extending from the periphery of the protective sleeve 12.

## Claims

1. A disposable safety syringe comprising:
a) a barrel (1),
b) an injection needle (4) that is attached to a needle-carrying member (3) disengageably fitted in, and/or on, the fore end of the barrel (1),
c) a plunger (6) that is slidable in the barrel (1) from an utmost retracted syringe-filling position to a forwardmost syringe-emptying position, and is fitted with a manually drivable stem (7) protruding from the rear end of the barrel (1),
d) a protective sleeve (12) that is longitudinally slidably fitted on the outside of the barrel (1), so as to be movable from a retracted rest position in which the needle (4) is exposed, into an advanced safety position in which the protective sleeve (12) extends all around the needle (4) so as to entirely cover the needle,
e) clamping means (16,17,16',17') comprising elastically flexible clamping tongues (16,16') and hook-like clamping teeth (17,17') that are provided at the rear free ends of said elastically flexible clamping tongues (16,16') extending in the longitudinal direction of the protective sleeve (12), and being made in one piece therewith, and the said clamping teeth (17,17') cooperate with a matching retaining rim on the rear end of barrel (1), the protective sleeve in its retracted position being fastened to the barrel (1), by said clamping teeth (17,17') engaged with said retaining rim,
f) releasing means for disengaging the clamping teeth (17,17') from the retaining rim on the rear end of the barrel (1), at the end, or almost at the end, of the forward stroke of the plunger (6), comprising a disc-shaped pusher member (19,19') that is secured to the rear end section extending out of the barrel (1) of the plunger stem (7) and co-operates with the clamping teeth (17,17'), the said releasing means thus unfastening the protective sleeve (12) from barrel (1) and allowing the protective sleeve to be moved in its advance safety position,
g) a spring (15) that is fitted between he barrel (1) and the protective sleeve (12), and is adapted for moving the protective sleeve (12) from its retracted rest position into its advanced safety position,
h) locking means (23,25) that are provided for automatically retaining the protective sleeve (12) in its advanced safety position, in a not retractil manner to the barrel (1),
characterized by the following features:
i) two diametrically opposite, slip back-preventing teeth (21) are provided at the rear end of barrel (1), which teeth can be caused to elastically diverge radially outwardly from each other, and have their facing inward sides formed with slanted abutment faces (22), the said slip back-preventing teeth (21) being caused to cooperate with the pusher member (19;19') secured to the rear end section of the stem (7) of plunger (6), in such a manner that the said teeth (21) when being elastically flexed in the outward direction, will allow the pusher member (19;19') to pass therebetween, and will be then engaged with the rear side of the pusher member (19;19'), so that the pusher member (19;19') becomes locked together with the stem (7) of plunger (6), in the forwardmost position of the plunger stroke, whereby any backward displacement of the pusher member (19;19') and the stem (7) is prevented,
j) the slip back-preventing teeth (21) are formed by means of cuts made in the sidewall of a boxlike head (10) which is integrally provided at the rear end of barrel (1), and the clamping tongues (16;16') protruding from the rear end of the protective sleeve (12), extend by their free ends formed with the hook-like clamping teeth (17;17') and slanted abutment faces (18;18'), into the said boxlike head (10), by being passed through respective apertures (20; 20', 44) formed in the bottom of the said head (10), the retaining rims at the rear end of barrel (1) being provided by the edges of said apertures with which the said clamping teeth (17, 17') are engaged, and the boxlike head (10) being formed with an opening at its rear side for the disc-shaped pusher member (19; 19') secured to the rear end section of the stem (7) of plunger (6), to get into the said head (10),
k) removable safety means (29; 34) are provided for closing at least partly the opening at the rear end of the boxlike head (10) and for preventing the pusher member (19, 19') from getting into the said head (10),
l) the locking means (23, 25) for automatically retaining the protective sleeve (12) in its advanced safety position to the barrel (1), lock the protective sleeve (12) in both senses with the needle-carrying member (3).

2. The syringe according to Claim 1, characterized in that two diametrically opposite clamping tongues (16) are provided with slanted abument faces (18) which are turned toward each other, and cooperate with the releasing pusher member (19).

3. The syringe according to Claim 1, characterized in that two pairs of diametrically opposite clamping tongues (16', 16') are provided, the tongues (16') of each pair having the slanted abutment faces (18') of the hook-like clamping teeth (17') turned outwardly in opposite directions, and being elastically drawable near to each other from their clamping position into their releasing position by the releasing pusher member (19'), which is secured to the rear end section of the stem (7) of plunger (6), and is formed with a recess (5) for each pair of tongues (16', 16'), into which the respective pair of tongues (16', 16') is axially insertable.

4. The syringe according to Claim 1, characterized in that the removable safety means are in form of a tearable strip (29) of paper or paperboard, which through a hole (30) and a radial cut (31) is threaded on the stem (7) of plunger (6) so as to be positioned between the pusher member (9) and the boxlike head (10) at the rear end of barrel (1), and is caused to cooperate with the rim of the opening at the rear end of said head (10).

5. The syringe according to Claim 1, characterized in that the removable safety means are in form of a cover member (34) which is provided with a radial cut (36) for the stem (7) of plunger (6) to be passed therethrough, and is applicable to the opening at the rear end of the boxlike head (10) of barrel (1) by means of extensions (35) which are fitted in the said head (10), and are caused to cooperate with the clamping tongues (16) so as to prevent the said tongues from being flexed into their releasing position.

6. The syringe according to Claim 1, characterized in that the protective sleeve (12) lying in its retracted rest position, axially bears backward against at least one stop member (11) provided on the rear end of barrel (1), and the said sleeve is formed at its rear end with at least two diametrically opposite tabs (13) radially extending from the periphery thereof, which have to be sustained by two fingers of a user's hand holding the syringe at the time of an injection.

7. The syringe according to Claim 1, characterized in that the locking means consist of at least one inward locking projection (25) in the protective sleeve (12), which is caused to cooperate with a respective outward projection in the needle-carrying member (3), and of at least one locking tongue (23) formed in the protective sleeve (12) by means of cuts made therein, and extending in the longitudinal direction thereof, the fore end of the said locking tongue (23) being integral with the protective sleeve (12), and the rear free end thereof tending to elastically flex radially inwardly, are caused to cooperate with a respective outward projection (203) in the needle-carrying member (3), the whole arrangement being such that with the protective sleeve (12) lying in its advanced safety position, the inward projection (5) for locking the said sleeve bears forwardly against the respective outward projection (303) in the needle-carrying member (3), and the rear free end of the locking tongue (23) bears backwardly against the respective outward projection (203) in the needle-carrying member (3).

8. The syringe according to Claim 7, characterized in that the protective sleeve (12) lying in its retracted rest position, bears by its retracted inward projection (27) against an outward projection (203) in the needle-carrying member (3), whereby the said sleeve (12) prevents the needle-carrying member (3) from being slipped off the fore end of barrel (1).

9. The syringe according to Claim 8, characterized in that the protective sleeve (12) lying in its retracted rest position, has its fore end arranged close to the rear end of a needle-covering cap (5) disengageably fitted on the fore end of the needle-carrying member (3), the inside diameter of the front hole (28) at the fore part of the protective sleeve (12) being tapered relative to the inside diameter of the rest of the said sleeve (12), and being only slightly greater than the outside diameter of the matching rear end portion of the needle-covering cap (5).

10. The syringe according to Claim 9, characterized in that the tapered front hole (28), and the retaining inward projection (27) in the protective sleeve (12), are formed by a tubular member (26; 26') fitted in and/or on the protective sleeve (12), and unremovably secured thereto.

11. The syringe according to Claim 1, characterized in that the stem (7) of plunger (6) is provided with an easily breakable weakened portion (32) at a location between the pusher member (19, 19') and the knob (9) on the rear end of stem (7).

## Patentansprüche

1. Sicherheitsspritze zum Einmalgebrauch mit
a) einem Zylinder (1),
b) einer Injektionsnadel (4), die an einem Nadelträger (3) befestigt ist, der lösbar in, und/oder an dem vorderen Ende des Zylinders (1) befestigt ist,
c) mit einem Kolben (6), der im Zylinder (1) gleitend vorgesehen ist, und zwar von einer zurückgezogenen Füllposition für die Spritze in eine vordere Entleerungsposition der Spritze, und der mit einem von Hand betätigbaren Schaft (7) versehen ist, der vom rückwärtigen Ende des Zylinders (1) vorsteht,
d) mit einer Schutzhülse (12), die in Längsrichtung gleitend an der Außenseite des Zylinders (1) vorgesehen ist derart, daß sie von einer zurückgezogenen Ruheposition, in der die Nadel (4) freiliegt, in eine vordere Sicherheitsposition bewegt werden kann, in der die Schutzhülse sich vollständig ringsum die Nadel (4) erstreckt, so daß sie die Nadel vollständig abdeckt,
e) mit Klemm-Mitteln (16,17,16',17') mit elastisch biegsamen Klemmzungen (16,16') und mit hakenförmigen Klemmzungen (17,17'), die an den rückwärtigen freien Enden der elastisch biegsamen Klemmzungen (16,16') vorgesehen sind, die sich in Längsrichtung der Schutzhülse (12) erstrecken, und die damit einstükkig hergestellt sind, wobei die Klemmzungen (17,17') mit einer passenden Halterille am rückwärtigen Ende des Zylinders (1) zusammenarbeiten, wobei die Schutzhülse in ihrer zurückgezogenen Position am Zylinder (1) befestigt ist, und zwar mit Hilfe der Klemmzungen (17,17'), die in die Halterille eingreifen,
f) mit Lösemitteln zum Lösen der Klemmzungen (17,17') von der Halterille am rückwärtigen Ende des Zylinders (1) am Ende oder nahezu am Ende des Vorwärtshubs des Kolbens (6), wobei die Lösemittel die folgenden Merkmale aufweisen: einen scheibenförmigen Schieber (19,19'), der an dem sich aus dem Zylinder (1) heraus erstreckenden rückwärtigen Endabschnitt des Schaftes (7) befestigt ist und der mit den Klemmzungen (17,17') zusammenarbeitet, wobei die Lösemittel die Schutzhülse (12) somit vom Zylinder (1) lösen und es ermöglichen, daß die Schutzhülse in ihre vordere Sicherheitslage bewegt wird,
g) mit einer Feder (15), die zwischen dem Zylinder (1) und der Schutzhülse (12) vorgesehen ist, und die die Schutzhülse (12) von der zurückgezogenen Ruhelage in die vordere Sicherheitslage bewegen kann,
h) mit einer Verriegelung (23,25), die vorgesehen ist, um die Schutzhülse (12) automatisch in der vorderen Sicherheitslage zu halten, und zwar in einer in Bezug auf den Zylinder (1) nicht einziehbaren Weise,
**dadurch gekennzeichnet,**
i) daß zwei einander diametral gegenüberliegend angeordnete Zähne (21) am rückwärtigen Ende des Zylinders (1) vorgesehen sind, die ein Zurückgleiten verhindern, die elastisch voneinander radial nach außen divergieren können, und deren nach innen weisende Seiten mit geneigten Anlageflächen (22) versehen sind, wobei die das Zurückgleiten verhindernden Zähne (21) mit dem Schieber (19;19') zusammenarbeiten, der am rückwärtigen Abschnitt des Schaftes (7) des Kolbens (6) befestigt ist, so daß die Zähne (21), wenn sie elastisch nach außen gebogen sind, es ermöglichen, daß der Schieber (19;19') dazwischen hindurchgeht, und dann an der rückwärtigen Seite des Schiebers (19;19') angreifen, so daß der Schieber (19;19') zusammen mit dem Schaft (7) des Kolbens (6) verriegelt wird, und zwar in der vordersten Lage des Hubes des Kolbens, wodurch jede Verschiebung des Schiebers (19;19') und des Schaftes (7) nach hinten verhindert wird,
j) daß die das Zurückgleiten verhindernden Zähne (21) mit Hilfe von Schnitten hergestellt sind, die in der Seitenwand eines kastenförmigen Kopfes (10) angelegt sind, der einstückig am rückwärtigen Ende des Zylinders (1) vorgesehen ist, wobei die Klemmzungen (16;16'), die aus dem rückwärtigen Ende der Schutzhülse (12) vorstehen, sich mit Hilfe ihrer freien Enden, die mit den hakenförmigen Klemmzungen (17;17') und den geneigten Anlageflächen (18;18') geformt sind, in den kastenförmigen Kopf (10) erstrecken, indem sie durch jeweilige Öffnungen (20;20,44) gehen, die im Boden des Kopfes (10) geformt sind, wobei die Halterillen am rückwärtigen Ende des Zylinders durch die Kanten der Öffnungen vorgesehen sind, mit denen die Klemmzähne (17,17') zusammenarbeiten, wobei der kastenförmige Kopf (10) eine Öffnung an seiner rückwärtigen Seite für den scheibenförmigen Schieber (19;19') hat, der am rückwärtigen Abschnitt des Schaftes (7) des Kolbens (6) befestigt ist, um in den Kopf (10) zu gelangen,
k) daß abnehmbare Sicherheitsmittel (29;34) vorgesehen sind, um wenigstens teilweise die Öffnung am rückwärtigen Ende des kastenförmigen Kopfes (10) zu verschließen und um zu verhindern, daß der Schieber (19,19') in den Kopf (10) eindringt,
l) und daß das Verriegelungsmittel (23,25), mit dem die Schutzhülse (12) automatisch in ihrer vorderen Sicherheitslage am Zylinder (1) gehalten wird, die Schutzhülse (12) in beiden Richtungen am Nadelträger verriegelt.

2. Sicherheitsspritze nach Anspruch 1,
**dadurch gekennzeichnet,**
daß zwei diametral einander gegenüberliegende Klemmmzungen (16) mit geneigten Anlageflächen (18) vorgesehen sind, die einander zugewandt angeordnet sind, und die mit dem auslösenden Schieber (19) zusammenarbeiten.

3. Sicherheitsspritze nach Anspruch 1,
**dadurch gekennzeichnet,**
daß zwei Paar einander diametral gegenüberliegender Klemmzungen (16,16') vorgesehen sind, wobei die Zungen (16') eines jeden Paares, die die geneigten Anlageflächen (18') der hakenförmigen Klemmzähne (17') haben, nach außen in gegenüberliegende Richtungen gedreht sind, und elastisch einander benachbart gezogen werden können, und zwar von ihrer Klemmposition in ihre Löseposition mit Hilfe des lösenden Schiebers (19'), der am rückwärtigen Endabschnitt des Schaftes des Kolbens befestigt ist, und der mit einer Aufnahme (5) für jedes Zungenpaar (16,16') geformt ist in die das betreffende Zungenpaar (16',16') in axialer Richtung eingesetzt werden kann.

4. Sicherheitsspritze nach Anspruch 1,
**dadurch gekennzeichnet,**
daß das entfernbare Sicherheitsmittel in Form eines abziehbaren Streifens (29) aus Papier oder Papierwerkstoff vorgesehen ist, wobei ein Durchgangsloch (30) und ein radialer Einschnitt (31) am Schaft (7) des Kolbens (6) vorgesehen sind, so daß sie zwischen dem Schieber (9) und dem kastenförmigen Kopf (10) am rückwärtigen Ende des Zylinders (1) angeordnet sind, und wobei das Mittel mit der Rille der Öffnung am rückwärtigen Ende des Kopfes (10) zusammenarbeitet.

5. Sicherheitsspritze nach Anspruch 1,
**dadurch gekennzeichnet,**
daß das entfernbare Sicherheitsmittel in Form einer Abdeckung (34) vorgesehen ist, die einen radialen Einschnitt (36) für den Schaft (7) des Kolbens (6) hat, der dort hindurchgeht, und an die Öffnung des rückwärtigen Endes des kastenförmigen Kopfes (10) des Zylinders (1) mit Hilfe von Fortsätzen (35) angewendet werden kann, die in den Kopf (10) eingepaßt sind, und die mit den Klemmzungen (16) zusammenarbeiten, so daß verhindert wird, daß die Zungen in ihre Löseposition verbogen werden.

6. Sicherheitsspritze nach Anspruch 1,
**dadurch gekennzeichnet,**
daß die Schutzhülse (12), wenn sie sich in ihrer zurückgezogenen Ruhelage befindet, axial nach hinten an wenigstens einem Anschlag (12) anliegt, der am rückwärtigen Ende des Zylinders (1) vorgesehen ist, und daß die Schutzhülse an ihrem rückwärtigen Ende mit wenigstens zwei diametral gegenüberliegenden Laschen (13) geformt ist, die sich von dessen Umfang in radialer Richtung erstrecken, und die von zwei Fingern der Hand des Benutzers gegriffen werden können, der die Spritze zwecks Injektion hält.

7. Sicherheitsspritze nach Anspruch 1,
**dadurch gekennzeichnet,**
daß das Verriegelungsmittel aus wenigstens einem inneren Verriegelungsvorsprung (25) in der Schutzhülse (12) besteht, der mit einem entsprechenden nach außen gerichteten Vorsprung im Nadelträger (3) zusammenarbeitet und aus wenigstens einer Verriegelungszunge (23), die in der Schutzhülse (12) mit Hilfe von darin angelegten Schnitten geformt ist, und die sich in deren Längsrichtung erstreckt, wobei das vordere Ende der Verriegelungszunge (23) einstückig mit der Schutzhülse (12) ist und das rückwärtige Ende des Teils dazu neigt, radial nach innen elastisch verbogen zu werden, und mit einer jeweiligen nach außen weisenden Projektion (203) im Nadelträger (3) zusammenarbeitet, wobei die gesamte Anordnung so ausgeführt ist, daß, wenn sich die Schutzhülse (12) in ihrer vorderen Sicherheitslage befindet, daß dann der innere Vorsprung (5) zum Verriegeln der Hülse nach vorne weist und sich an die jeweilige nach außen weisende Projektion (303) im Nadelträger anlegt, und daß das rückwärtige freie Ende der Verriegelungszunge (23) an der jeweiligen äußeren Projektion (203) im Nadelträger (3) anliegt.

8. Sicherheitsspritze nach Anspruch 7,
**dadurch gekennzeichnet,**
daß die Schutzhülse (12) in ihrer zurückgezogenen Ruhelage mit Hilfe ihrer zurückgezogenen inneren Projektion (27) an einer äußeren Projektion (203) im Nadelträger (3) anliegt, wobei die Schutzhülse verhindert, daß der Nadelträger (3) aus dem vorderen Ende des Zylinders (1) austritt.

9. Sicherheitsspritze nach Anspruch 8,
**dadurch gekennzeichnet,**
daß das vordere Ende der Schutzhülse (12), wenn diese sich in ihrer zurückgezogenen Ruhelage befindet, eng benachbart dem rückwärtigen Ende der die Nadel abdeckenden Kappe (5) befindet, die lösbar am vorderen Ende des Nadelträgers (3) befestigt ist, wobei der Innendurchmesser des vorderen Lochs (28) im vorderen Teil der Schutzhülse (12) relativ zum Innendurchmesser des Restes der Schutzhülse (12) sich verjüngt und nur etwas größer ist als der Außendurchmesser des passenden rückwärtigen Endteils der die Nadel schützenden Kappe (5).

10. Sicherheitsspritze nach Anspruch 9,
**dadurch gekennzeichnet,**
daß das sich verjüngende vordere Loch (28) und der haltende, innere Vorsprung (27) in der Schutzhülse (12) mit Hilfe eines rohrförmigen Gliedes (26;26') geformt sind, das in und/oder an der Schutzhülse (12) geformt ist, und das damit unlösbar verbunden ist.

11. Sicherheitsspritze nach Anspruch 1,
**dadurch gekennzeichnet,**
daß der Schaft (7) des Kolbens (6) mit einer Sollbruchstelle (32) versehen ist, und zwar zwischen dem Schieber (19,19') und dem Knopf (9) am rückwärtigen Ende des Schaftes (7).

## Revendications

1. Seringue de sécurité à usage unique comprenant :
a) un cylindre (1),
b) une aiguille pour injection (4) qui est fixée à un élément de support de l'aiguille (3) fixé de manière amovible dans et / ou sur l'extrémité avant du cylindre (1),
c) un piston (6) qui peut coulisser dans le cylindre (1) depuis une position de remplissage de la seringue entièrement rétractée vers une position de vidage de la seringue entièrement avancée et qui est muni d'une tige (7) entraînée manuellement faisant saillie depuis l'extrémité arrière du cylindre (1),
d) un manchon protecteur (12) fixé de manière coulissante longitudinalement sur l'extérieur du cylindre (1), de manière à pouvoir se déplacer depuis une position de repos rétractée dans laquelle l'aiguille (4) est exposée vers une position de sécurité avancée dans laquelle le manchon protecteur (12) s'étend tout autour de l'aiguille (4), de manière à recouvrir entièrement l'aiguille,
e) des moyens de fixation (16, 17, 16', 17') comprenant des langues de fixation flexibles de manière élastique (16, 16') et des dents de fixation en forme de crochet (17, 17') qui sont disposées sur les extrémités libres arrières desdites langues de fixation flexibles de manière élastique (16, 16') s'étendant dans le sens longitudinal du manchon protecteur (12) et faites d'une seule pièce avec celles-ci, et lesdites dents de fixation (17, 17') coopèrent avec un rebord de retenue correspondant sur l'extrémité arrière du cylindre (1), le manchon protecteur en sa position rétractée étant fixé au cylindre (1) au moyen desdites dents de fixation (17, 17') engagées avec ledit rebord de retenue,
f) des moyens de relâchement destinés à désengager les dents de fixation (17, 17') du rebord de retenue sur l'extrémité arrière du cylindre (1) à la fin ou presque de la course avant du piston (6), comprenant un élément de poussoir en forme de disque (19, 19') qui est fixé à la section terminale arrière s'étendant hors du cylindre (1) de la tige (7) du piston et qui coopère avec les dents de fixation (17, 17'), lesdits moyens de relâchement desserrant ainsi le manchon protecteur (12) du cylindre (1) et permettant au manchon protecteur d'être déplacé dans sa position de sécurité avancée,
g) un ressort ( 15) qui est fixé entre le cylindre (1) et le manchon protecteur (12) et qui est destiné à déplacer le manchon protecteur (12) depuis sa position de repos rétractée vers sa position de sécurité avancée,
h) des moyens de verrouillage (23, 25) qui sont destinés à retenir automatiquement le manchon protecteur (12) dans sa position de sécurité avancée, de manière non rétractile par rapport au cylindre (1),
caractérisé par les caractéristiques suivantes :
deux dents anti-retour diamétralement opposées (21) sont disposées à l'extrémité arrière du cylindre (1), lesdites dents peuvent être obligées à dévier de manière élastique radialement vers l'extérieur l'une par rapport à l'autre et leurs côtés tournés vers l'intérieur sont formés avec des faces de butée inclinées (22), lesdites dents anti-retour (21) étant obligées de coopérer avec l'élément de poussoir (19, 19') fixé à la section terminale arrière de la tige (7) du piston (6), de telle sorte que lesdites dents (21), lorsqu'elles sont fléchies de manière élastique dans le sens extérieur, permettent à l'élément de poussoir (19, 19') de passer entre celles-ci et s'engagent alors avec le côté arrière de l'élément de poussoir (19, 19'), de sorte que l'élément de poussoir (19, 19') est verrouillé avec la tige (7) du piston (6), dans la position entièrement avancée de la course du piston, tout déplacement vers l'arrière de l'élément de poussoir (19, 19') et de la tige (7) étant de ce fait empêché,
les dents anti-retour (21) sont formées au moyen de découpes faites dans la paroi latérale d'une tête (10) en forme de boîtier qui est intégralement fournie à l'extrémité arrière du cylindre (1), et les langues de fixation (16, 16') faisant saillie depuis l'extrémité arrière du manchon protecteur (12) s'étendent en leurs extrémités libres formées avec les dents de fixation en forme de crochet (17,17') et les faces de butée incinées (18, 18') dans ladite tête en forme de boîtier (10), en étant passées à travers des ouvertures respectives (20, 20', 44) formées dans le fond de ladite tête (10), les rebords de retenue à l'extrémité arrière du cylindre (1) étant fournis par les arêtes desdites ouvertures avec lesquelles lesdites dents de fixation (17, 17') sont engagées, et la tête (10) en forme de boîtier étant formée avec une ouverture dans son côté arrière pour l'élément de poussoir en forme de disque (19, 19') fixé à la section terminale arrière de la tige (7) du piston (6), de manière à entrer dans ladite tête (10),
des moyens de sécurité amovibles (29, 34) sont destinés à fermer au moins partiellement l'ouverture à l'extrémité arrière de la tête en forme de boîtier (10) et à empêcher l'élément de poussoir (19, 19') d'entrer dans ladite tête (10),
les moyens de verrouillage (23, 25) destinés à retenir automatiquement le manchon protecteur (12) en sa position de sécurité avancée par rapport au cylindre (1) verrouillent le manchon protecteur (12) dans les deux sens avec l'élément de support de l'aiguille (3).

2. Seringue selon la revendication 1, caractérisée en ce que deux langues de fixation (16) diamétralement opposées sont munies de faces de butée inclinées qui sont tournées l'une vers l'autre et coopèrent avec l'élément de poussoir (19) de relâchement.

3. Seringue selon la revendication 1, caractérisée en ce que deux paires de langues de fixation (16, 16') diamétralement opposées sont fournies, les langues (16') de chaque paire présentant les faces de butée inclinées (18') des dents de fixation en forme de crochet (17') tournées vers l'extérieur en des directions opposées, et pouvant être poussées de manière élastique l'une vers l'autre depuis leur position de fixation vers leur position de relâchement par l'élément de poussoir de relâchement (19') qui est fixé à la section terminale arrière de la tige (7) du piston (6) et est formé avec un évidement (5) pour chaque paire de langues (16', 16') dans lequel la paire de langues respectives (16', 16') peut être insérée de manière axiale.

4. Seringue selon la revendication 1, caractérisée en ce que les moyens de sécurité amovibles ont la forme d'une bande (29) de papier ou de carton pouvant être déchirée qui, à travers un trou (30) et une découpe radiale (31), est filetée sur la tige (7) du piston (6) de manière à être placée entre l'élément de poussoir (9) et la tête en forme de boîtier (10) à l'extrémité arrière du cylindre (1), et qui doit coopérer avec le rebord de l'ouverture à l'extrémité arrière de ladite tête (10).

5. Seringue selon la revendication 1, caractérisée en ce que les moyens de sécurité amovibles ont la forme d'un élément de couvercle (34) qui est muni d'une découpe radiale (36) pour la tige (7) du piston (6) devant passer à travers celle-ci et qui peut s'appliquer à l'ouverture à l'extrémité arrière de la tête en forme de boîtier (10) du cylindre (1) au moyen d'extensions (35) qui sont fixées dans ladite tête (10) et sont obligées de coopérer avec les langues de fixation (16) de manière à empêcher lesdites langues d'être fléchies dans leur position de relâchement.

6. Seringue selon la revendication 1, caractérisée en ce que le manchon protecteur (12) dans sa position de repos rétractée repose axialement vers l'arrière contre au moins un élément d'arrêt (11) disposé sur l'extrémité arrière du cylindre (1), et ledit manchon est formé en son extrémité arrière d'au moins deux pattes diamétralement opposées (13) s'étendant radialement depuis la périphérie de celui-ci, qui doivent être soutenues par deux doigts de la main de l'utilisateur tenant la seringue au moment de l'injection.

7. Seringue selon la revendication 1, caractérisée en ce que les moyens de verrouillage consistent an moins en une saillie de verrouillage vers l'intérieur (25) dans le manchon protecteur (12), qui est obligée de coopérer avec une saillie vers l'extérieur respective dans l'élément de support de l'aiguille (3), et d'au moins une langue de verrouillage (23) formée dans le manchon protecteur (12) au moyen de découpes faites dans celui-ci et s'étendant dans le sens longitudinal de celui-ci, l'extrémité avant de ladite langue de verrouillage (23) étant intégrée au manchon protecteur (12) et l'extrémité libre arrière de celle-ci tendant à fléchir de manière élastique radialement vers l'intérieur sont obligées de coopérer avec une saillie vers l'extérieur respective (203) dans l'élément de support de l'aiguille (3), tout le dispositif étant tel que, avec le manchon protecteur (12) se trouvant dans sa position de sécurité avancée, la saillie vers l'avant (5) destinée à verrouiller ledit manchon repose vers l'avant contre une saillie vers l'extérieur respective (303) dans l'élément de support de l'aiguille (3), et l'extrémité libre arrière de la langue de verrouillage (23) repose vers l'arrière contre la saillie vers l'extérieur respective (203) dans l'élément de support de l'aiguille (3).

8. Seringue selon la revendication 7, caractérisée en ce que le manchon protecteur (12) se trouvant dans sa position de repos rétractée repose par sa saillie vers l'intérieur rétractée (27) contre une saillie vers l'extérieur (203) dans l'élément de support de l'aiguille (3), ledit manchon (12) empêchant de ce fait l'élément de support de l'aiguille (3) de glisser hors de l'extrémité avant du cylindre (1).

9. Seringue selon la revendication 8, caractérisée en ce que le manchon protecteur (12) se trouvant dans sa position de repos rétractée présente son extrémité avant disposée à côté de l'extrémité arrière du couvercle recouvrant l'aiguille (5) fixé de manière amovible sur l'extrémité avant de l'élément de support de l'aiguille (3), le diamètre interne du trou avant (28) sur la partie avant du manchon protecteur (12) étant conique par rapport au diamètre interne du reste dudit manchon (12) et étant seulement légèrement plus large que le diamètre externe de la portion terminale arrière correspondante du couvercle recouvrant l'aiguille (5).

10. Seringue selon la revendication 9, caractérisée en ce que le trou avant conique (28) et la saillie vers l'intérieur de retenue (27) dans le manchon protecteur (12) sont formés par un élément tubulaire (26, 26') fixé dans et / ou sur le manchon protecteur (12) et fixé de manière inamovible à celui-ci.

11. Seringue selon la revendication 1, caractérisée en ce que la tige (7) du piston (6) est munie d'une portion affaiblie (32) pouvant facilement se briser en un lieu entre l'élément de poussoir (19, 19') et le bouton (9) sur l'extrémité arrière de la tige (7).
